# EUROPEAN PATENT APPLICATION

(11) **EP 4 427 692 A1**
(43) Date of publication of application: **11.09.2024**
(21) Application number: 22889967.0
(22) Date of filing: 02.11.2022
(51) Int. Cl.: A61B 18/12, A61M 25/10

(54) **ABLATION CATHETER SYSTEM**

(30) Priority: 04.11.2021 JP 2021179993
(71) Applicant: Toray Industries, Inc., Tokyo 103-8666 (JP)
(72) Inventor: WATANABE, Akio, Otsu-shi, Shiga 520-2141 (JP)
(74) Representative: Kador & Partner Part mbB
(86) International application number: PCT/JP2022/040956
(87) International publication number: WO 2023/080147

(57) **Abstract**

The present invention aims to provide a balloon ablation catheter system that estimates a positional relationship between a balloon and a shaft while executing time-stable temperature control and informs a practitioner of the positional relationship. The present invention provides the ablation catheter system including: the shaft having a lumen through which fluid can be delivered; the balloon attached to the distal end of the shaft; heating means arranged in the balloon; heating power supply means for supplying power to the heating means; a temperature sensor arranged near the heating means; and a control section for estimating the positional relationship between the balloon and the shaft. The control section calculates an actual value of a time fluctuation in output power from the heating power supply means or an actual value of a time fluctuation in a temperature by the temperature sensor, and estimates the positional relationship between the balloon and the shaft from the obtained actual value.

## Description

### TECHNICAL FIELD

The present invention relates to an ablation catheter system used for medical applications.

### BACKGROUND ART

Catheter ablation treatment is a method for treating medical conditions such as arrhythmia caused by atrial fibrillation, endometriosis, and cancer by inserting a catheter into a body and heating the distal end of the catheter to destroy a target site or by another method.

Various catheters are developed as ablation catheters for use in this treatment procedure and known to include: a catheter in which a heating electrode at the distal end of the catheter is placed in close contact with the target site and destroys the target site by heating with a radiofrequency current; and an ablation catheter with a balloon in which the balloon is attached to the distal end of the catheter and fluid in the balloon is heated using a radiofrequency to destroy myocardial cells causing arrhythmia for treatment of arrhythmia.

In order to effectively perform the catheter ablation treatment, it is important that the temperature of the heated site promptly reach a temperature at which target myocardial tissue is destroyed and that such a temperature is stably maintained even under an influence of changing environment of the myocardial tissue, a surrounding blood flow, a body fluid, or the like. The existing technique also proposes a method for stably and efficiently increasing the temperature of a control target to a target temperature and maintaining the temperature of the control target at the target temperature by changing the temperature of the heating body appropriately and promptly in a situation where the temperature of the control target varies.

In Patent Document 1, a balloon ablation catheter system is described in which a radiofrequency power supply coil electrode is formed in the balloon, a thermocouple temperature sensor is formed at a contact point with the radiofrequency power supply coil, and a balloon temperature is controlled by using a temperature signal from a thermocouple as an manipulated variable.

In addition, Patent Document 2 describes a method in which a balloon ablation catheter system has a plurality of heating electrodes, each of which is in contact with the treatment target tissue, on a balloon surface. In the method, when the temperature of each of the electrodes exceeds a selected maximum temperature, the radiofrequency power supplied to one of the plural heating electrodes is suppressed, so as to be able to always apply an optimal temperature for ablation to the treatment target tissue.

### PRIOR ART DOCUMENTS

### PATENT DOCUMENTS

[Patent Document 1] JP 2012-254140 A
[Patent Document 2] JP 2019-13759 A

### SUMMARY OF THE INVENTION

### PROBLEMS TO BE SOLVED BY THE INVENTION

It is described in Patent Document 1 that, in the balloon ablation catheter system, the temperature near the radiofrequency power supply coil electrode is measured, and the balloon surface temperature is controlled on the basis of the measured temperature. However, depending on a balloon shape or a positional relationship with the ablation target tissue, the temperature near the coil electrode is inadequate as the balloon surface temperature. Thus, there is a problem that the balloon surface temperature cannot be controlled adequately. That is, the temperature applied to the treatment target tissue cannot always be controlled accurately.

A discrepancy between the coil electrode temperature and the balloon surface temperature varies significantly depending on the positional relationship between the balloon and the shaft, but there is a problem of not having means for communicating this point to a practitioner.

In Patent Document 2, the balloon ablation catheter system capable of always applying the optimal temperature for the ablation to the treatment target tissue can be provided. However, it is required to form the plurality of the heating electrodes on the balloon surface by vapor deposition, screen printing, inkjet coating, or the like, or it is required to perform a process of adhering a highly conductive metallic foil, which is produced in advance, to the balloon surface. Thus, there is a problem of extremely high manufacturing cost and extreme difficulty in manufacturing. In addition, the heating electrode formed on the balloon surface, which repeatedly expands and contracts, has many durability issues in actual use.

Furthermore, while a plurality of balloon surface temperature sensors can measure a plurality of the temperatures on the balloon surface regardless of a contact state with the tissue, there is no means for communicating information on the positional relationship between the balloon and the shaft to the practitioner. Thus, there is a problem that, when pressing the balloon against the tissue, the practitioner cannot apply an intended pressing pressure to the tissue.

### MEANS FOR SOLVING THE PROBLEMS

In order to solve the above problem, the present inventors intensively studied to discover the following inventions (1) to (6):
(1) An ablation catheter system, comprising:
   a shaft having a lumen through which fluid can be delivered;
   a balloon attached to the distal end of the shaft;
   heating means arranged in the balloon;
   heating power supply means for supplying power to the heating means;
   a temperature sensor arranged near the heating means; and
   a control section for estimating a positional relationship between the balloon and the shaft,
   wherein the control section calculates an actual value of a time fluctuation in output power from the heating power supply means or an actual value of a time fluctuation in a temperature by the temperature sensor, and estimates the positional relationship between the balloon and the shaft from the obtained actual value of the time fluctuation in the output power and the obtained actual value of the time fluctuation in the temperature by the temperature sensor.
(2) The ablation catheter system of (1), comprising a recording section for recording a threshold of the time fluctuation in the output power from the heating power supply means or a threshold of the time fluctuation in the temperature by the temperature sensor,
   wherein the control section compares the threshold of the time fluctuation and the actual value of the time fluctuation in a predetermined cycle to estimate the positional relationship between the balloon and the shaft.
(3) The ablation catheter system of (1), comprising a recording section for recording a conversion table used to convert the actual value of the time fluctuation in the output power from the heating power supply means or the time fluctuation in the temperature by the temperature sensor into the positional relationship between the balloon and the shaft,
   wherein the control section estimates the positional relationship between the balloon and the shaft on the basis of the time fluctuation and the conversion table.
(4) The ablation catheter system of any one of (1) to (3), comprising a display section for displaying estimated information on the positional relationship between the balloon and the shaft.
(5) The ablation catheter system of (4), wherein the display section displays the positional relationship between the balloon and the shaft with a collection of surface light-emitting elements.
(6) An ablation catheter system, comprising:
   a shaft having a lumen through which fluid can be delivered;
   a balloon attached to the distal end of the shaft;
   heating means arranged in the balloon;
   heating power supply means for supplying power to the heating means;
   a temperature sensor arranged near the heating means;
   a control section for calculating an actual value of a time fluctuation in output power from the heating power supply means or an actual value of a time fluctuation in a temperature by the temperature sensor; and
   a display section for outputting the actual value of the time fluctuation.

### EFFECT OF THE INVENTION

The present invention can provide the system capable of estimating the positional relationship between the balloon and the shaft, which is important in ablation treatment, by controlling the temperature of the balloon while detecting the temperature of the balloon in contact with treatment target tissue, and by obtaining the actual value of the time fluctuation in the output power from the heating power supply means or the time fluctuation in the temperature by the temperature sensor.

### BRIEF DESCRIPTION OF THE DRAWINGS

Fig. 1 is a view illustrating external appearance of an ablation catheter system according to a first embodiment.
Fig. 2 is an explanatory view of a balloon catheter according to the first embodiment.
Fig. 3 is a block diagram of a temperature control system implemented by heating power supply means.
Fig. 4 is a flowchart illustrating temperature control operation of the ablation catheter system according to the first embodiment.
Fig. 5 is a view illustrating external appearance of an experimental system simulating actual use of the ablation catheter system.
Fig. 6 is a schematic view for explaining a relationship between a balloon and a shaft in a coaxial state.
Fig. 7 is a schematic view for explaining the relationship between the balloon and the shaft in an intermediate range state.
Fig. 8 is a schematic view for explaining the relationship between the balloon and the shaft in a non-coaxial state.
Fig. 9 indicates actual measured values of a temperature by a temperature sensor 15 for a coil electrode, a temperature by a lumen temperature sensor 5, and output power of the heating power supply means in cases where the temperature by the temperature sensor 15 for the coil electrode (a temperature near a heating coil electrode) is controlled to be 70°C during ablation operation in the coaxial state.
Fig. 10 indicates the actual measured values of the temperature by the temperature sensor 15 for the coil electrode, the temperature by the lumen temperature sensor 5, and the output power of the heating power supply means in cases where the temperature by the temperature sensor 15 for the coil electrode (the temperature near the heating coil electrode) is controlled to be 70°C during the ablation operation in the non-coaxial state.
Fig. 11 is a schematic view illustrating a flow of fluid in the balloon in the coaxial state.
Fig. 12 is a schematic view illustrating the flow of the fluid in the balloon in the non-coaxial state.
Fig. 13 indicates the temperature by the temperature sensor 15 for the coil electrode, the temperature by the lumen temperature sensor 5, and the actual measured value of the output power of the heating power supply means in cases where the state is changed from the non-coaxial state to the coaxial state and, after about 30 seconds, further changed from the coaxial state to the non-coaxial state during operation in which the temperature by the temperature sensor 15 for the coil electrode (the temperature near the heating coil electrode) is controlled to be 70°C.
Fig. 14 is a flowchart of a program for estimating the coaxial state, the intermediate range state, or the non-coaxial state in the first embodiment.
Fig. 15 is a view illustrating external appearance of the heating power supply means in the first embodiment.
Fig. 16 is a view illustrating external appearance of heating power supply means in a second embodiment.
Fig. 17 is a flowchart of a program for estimating the coaxial state, the intermediate range state, or the non-coaxial state in the second embodiment.
Fig. 18 is a view illustrating external appearance of heating power supply means in a third embodiment.
Fig. 19 is a flowchart of a program for estimating the coaxial state, the intermediate range state, or the non-coaxial state in the third embodiment.
Fig. 20 is a view illustrating a configuration of a display for the coaxial state, the intermediate range state, and the non-coaxial state in the third embodiment.
Fig. 21 illustrates an example of the display in which a white surface light-emitting LED and a color filter are combined.
Fig. 22 is a flowchart of a program for estimating the coaxial state, the intermediate range state, or the non-coaxial state in a fourth embodiment.
Fig. 23 is a flowchart in which a program for estimating the positional relationship between the balloon and the shaft is added to a flowchart of an overall control system for the heating power supply means.
Fig. 24 is a graph indicating a calculation result of a standard deviation of the output power in a fifth embodiment.
Fig. 25 is a flowchart of a program for estimating the coaxial state or the non-coaxial state in the fifth embodiment.
Fig. 26 is a view illustrating external appearance of heating power supply means in a sixth embodiment.

### MODE FOR CARRYING OUT THE INVENTION

An ablation catheter system according to the present invention includes: a shaft having a lumen through which fluid can be delivered; a balloon attached to the distal end of the shaft; heating means arranged in the balloon; heating power supply means for supplying power to the heating means; a temperature sensor arranged near the heating means; and a control section for estimating a positional relationship between the balloon and the shaft. The control section calculates an actual value of a time fluctuation in output power from the heating power supply means or an actual value of a time fluctuation in a temperature by the temperature sensor, and estimates the positional relationship between the balloon and the shaft from the obtained actual value.

Preferred embodiments of the present invention are described in detail below with reference to the drawings. However, the present invention is not limited to these modes. The same reference numerals are used for the same elements, and thus the overlapping description is not made. In addition, proportions in the drawings do not necessarily correspond to those in the description.

### (First Embodiment)

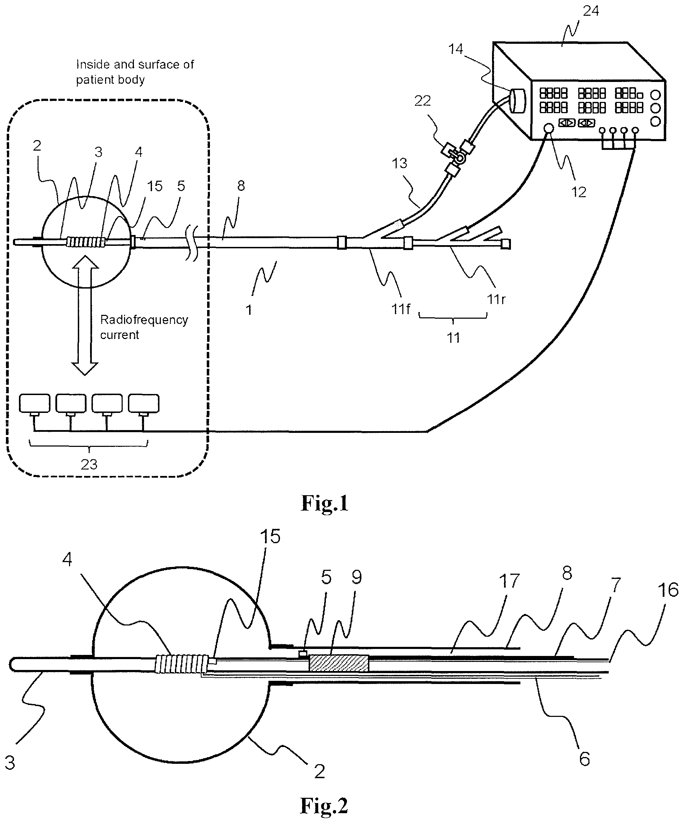

Fig. 1 is a view illustrating external appearance of an ablation catheter system according to a first embodiment. In Fig. 1, a control section (not illustrated) provided in heating power supply means 24 calculates an manipulated variable on the basis of a temperature by a temperature sensor 15 for a coil electrode that is arranged near a heating coil electrode 4 as heating means, and controls the heating means on the basis of the manipulated variable such that a balloon surface reaches a set target temperature. A recording section (not illustrated) provided in the heating power supply means 24 records, as a threshold that is required for a determination by the control section and set in advance, a threshold of a time fluctuation in output power of the heating power supply means 24. In the first embodiment, the heating power supply means 24 has the control section. However, the heating power supply means 24 may have an additional control section, or may further have a remote control section via the Internet or the like.

Here, the control section compares the threshold of the time fluctuation in the output power of the heating power supply means 24, which is recorded in the recording section, and a value of the time fluctuation in the output power of the heating power supply means 24 in a predetermined cycle, and thereby estimates a positional relationship between a balloon 2 and an outer cylinder shaft 8. As embodiments, which will be described below, the recording section provided in the heating power supply means 23 may record, as the threshold that is required for the determination by the control section and is set in advance, a threshold of a time fluctuation in the temperature by the temperature sensor 15 for the coil electrode, and the control section may estimate the positional relationship between the balloon 2 and the outer cylinder shaft 8 by comparing, in a predetermined cycle, a threshold of the time fluctuation in the temperature measured by the temperature sensor 15 for the coil electrode, which is recorded in the recording section, and an actual value of the time fluctuation in the temperature by the temperature sensor 15 for the coil electrode.

Here, the time fluctuation in the output power of the heating power supply means 24 refers to a value of the output power that changes over a course of a treatment time, and the time fluctuation in the coil electrode temperature refers to a measured value by the coil electrode temperature sensor 15 that changes over the course of the treatment time. In the first embodiment, the same control section controls the heating means and estimates the positional relationship between the balloon and shaft. However, one of the control sections may control the heating means, and the other of the control sections may estimate the positional relationship between the balloon and the shaft.

As illustrated in detail in Fig. 2 that is an explanatory view of a balloon catheter according to the first embodiment, a balloon catheter 1 includes a double-tube shaft that has the outer cylinder shaft 8 and an inner cylinder shaft 3 inserted into a lumen of the outer cylinder shaft 8 and is structured to allow the outer cylinder shaft 8 and the inner cylinder shaft 3 to slide with respect to each other.

The outer cylinder shaft 8 and the inner cylinder shaft 3 each have a cylindrical structure with the lumen (space) inside the cylinder. Since the inner cylinder shaft 3 is inserted into the lumen of the outer cylinder shaft 8, a lumen 16 exists between the outer cylinder shaft 8 and the inner cylinder shaft 3. The double-tube shaft serves as a fluid channel through this lumen 16 between the outer cylinder shaft 8 and the inner cylinder shaft 3 and is structured to deliver fluid from a hand side into the balloon 2. Here, the fluid means fluid flowing inside the balloon 2, and examples of the fluid are liquids such as water and physiological saline and gas such as nitrogen gas.

It should be noted that a catheter shaft of the balloon catheter 1 may only be formed of the outer cylinder shaft 8 connected to the balloon 2. In this case, the space inside the outer cylinder shaft 8 communicates with the inner space of the balloon 2, which is formed of the single shaft only. In addition, a lumen temperature sensor 5 may be arranged in the lumen 16.

The outer cylinder shaft 8 and the inner cylinder shaft 3 are designed to be 500 mm to 1700 mm in length. However, the lengths of the outer cylinder shaft 8 and the inner cylinder shaft 3 are not particularly limited as long as the lengths thereof are optimal for ablation treatment.

A material for each of the outer cylinder shaft 8 and the inner cylinder shaft 3 is preferably a flexible material with an excellent antithrombotic property. Examples of the flexible material with the excellent antithrombotic property include, but are not limited to, fluoropolymers, polyamides, polyurethane-based polymers, and polyimides.

The outer cylinder shaft 8 is preferably formed of multiple layers of different flexible materials in order to simultaneously ensure slidability with respect to the inner cylinder shaft 3 and adhesiveness or heat-weldability to the balloon 2.

The outer cylinder shaft 8 is designed to have an outer diameter of 3.0 mm to 4.0 mm and an inner diameter of 2.5 mm to 3.5 mm. Meanwhile, the inner cylinder shaft 3 is designed to have an outer diameter of 1.4 mm to 1.7 mm and an inner diameter of 1.1 mm to 1.3 mm.

In the balloon catheter 1, the expandable/contractable balloon 2 is fixed to the distal end side of the double-tube shaft.

The balloon 2 preferably has a shape that can fit a blood vessel and, for example, preferably has a spherical shape with a diameter of 15 mm to 40 mm as a size that fits a pulmonary venous junction of the left atrium. Examples of the spherical shape are a true spherical shape, an oblate spherical shape, a prolate spherical shape, and a substantial spherical shape.

A film thickness of the balloon 2 is preferably 1 µm to 200 µm. In addition, a material for the balloon 2 is preferably an elastic material with the excellent antithrombotic property and, more preferably, a polyurethane-based polymer material or the like.

As illustrated in Fig. 2, the distal end portion of the balloon 2 is fixed to the distal end portion of the inner cylinder shaft 3 in the longitudinal direction, and the proximal end portion of the balloon 2 is fixed to the distal end portion of the outer cylinder shaft 8 in the longitudinal direction. Here, adhesion or heat welding is a preferred method for fixing the balloon 2 to the outer cylinder shaft 8 and the inner cylinder shaft 3.

The balloon 2 is fixed just as described. Accordingly, when the inner cylinder shaft 3 slides with respect to the outer cylinder shaft 8, the distal end side of the balloon 2 is pulled by the inner cylinder shaft 3 to stretch, but the proximal end side of the balloon 2 is fixed to the outer cylinder shaft 8 and thus does not move. As a result, the balloon 2 stretches or contracts in the longitudinal direction by the sliding motion of the inner cylinder shaft 3 and the outer cylinder shaft 8, thereby enabling to adjust a length of the balloon 2 in the longitudinal direction.

In the balloon 2, the heating coil electrode 4 that Joule-heats the nearby fluid by radiofrequency energization is arranged in a substantially central portion on the inner cylinder shaft 3. The temperature sensor 15 for the coil electrode that measures a temperature of the fluid near the heating coil electrode 4 is arranged near the heating coil electrode 4. Here, "near" refers to a position within a range where the fluid is Joule-heated by a radiofrequency of the heating coil electrode 4. In addition, the temperature sensor 15 for the coil electrode is preferably arranged in contact with the heating coil electrode 4.

As an arrangement mode of the temperature sensor 15 for the coil electrode, in cases where the coil electrode temperature sensor 15 is a thermocouple, a junction between conductive wires that are a radiofrequency power supply lead wire 6 and a coil electrode temperature sensor lead wire 16 forms the thermocouple and serves as the temperature sensor 15 for the coil electrode.

In a lumen 17 between the outer cylinder shaft 8 and the inner cylinder shaft 3, the lumen temperature sensor 5 for measuring the temperature of the fluid flowing through the lumen 17 is arranged. In the lumen 17, the lumen temperature sensor 5 is preferably arranged at a position of 0 to 200 mm, more preferably, 5 to 40 mm from the proximal end edge of the balloon, that is, from the distal end edge of the outer cylinder shaft 8. However, the above dimensions may be changed appropriately according to a cross-sectional area of the lumen 17 or a vibration amount of the fluid generated by vibration generation means.

As an arrangement mode of the lumen temperature sensor 5, it is preferred to weld the distal end portion of a lumen temperature sensor lead wire 7 (for example, formed of two copper and constantan wires that are insulated from each other by outer coating such as enamel coating), to fix the lumen temperature sensor 5, which is formed of a thermocouple, to the inner cylinder shaft 3 or the outer cylinder shaft 8 by holding means such as a shrinkable tube, an adhesive tape, or an adhesive, and to hold a heat-sensitive section of the lumen temperature sensor 5 open in the lumen 17.

Since the heat-sensitive section is held open in the lumen 17, the lumen temperature sensor 5 can detect the temperature of the fluid in the lumen 17 with high thermal responsiveness without deterioration of the responsiveness caused by the temperature of the inner cylinder shaft 3 or the outer cylinder shaft 8 having a large heat capacity.

It should be noted that the radiofrequency power supply lead wire 6 for supplying radiofrequency power is connected to the coil electrode 4 for the radiofrequency energization.

As illustrated in Figs. 1 and 2, the radiofrequency power supply lead wire 6, the lumen temperature sensor lead wire 7, and the electrode temperature sensor lead wire 16, which are inserted into the lumen 17, extend along the inner cylinder shaft 3 in the lumen 17 to the hand side of the balloon catheter 1 and are connected to a connector 12 via the inside of a handle 11 connected to the hand side.

The connector 12 has an excellent waterproof design capable of preventing improper connection, but a structure thereof is determined in consideration of convenience of a practitioner and a design matter. In addition, similar to the handle 11, any highly chemical-resistant material may be used to constitute the connector 12, and, for example, a polysulfone, a polyetherimide, a polycarbonate, or an ABS resin can be used.

The connector 12 has a high-conductivity metal pin therein. The radiofrequency power supply lead wire 6, the lumen temperature sensor lead wire 7, and the electrode temperature sensor lead wire 16 are connected to this high-conductivity metal pin and thereby connected to radiofrequency power supply means 23.

The material for the high-conductivity metal pin provided in the connector 12 can be of any type of high-conductivity metal. Examples of the high-conductivity metal are copper, silver, gold, platinum, and alloys thereof. The outside of the high-conductivity metal pin may be protected by any electrically-insulating, chemical-resistant material. For example, peaks, polysulfones, polyurethane-based polymers, polypropylenes, or polyvinyl chloride can be used as such a material.

A structure of the handle 11 is designed to be easily grasped and operated by an operator by hand. A material constituting the handle 11 is preferably a highly chemical-resistant material, and, for example, a polycarbonate or the ABS resin can be used.

The handle 11 includes a front handle 11f and a rear handle 11r that are slidable with respect to each other. The front handle 11f and the rear handle 11r each have a branch path, and the connector 12 is connected to one of the branch paths of the rear handle 11r.

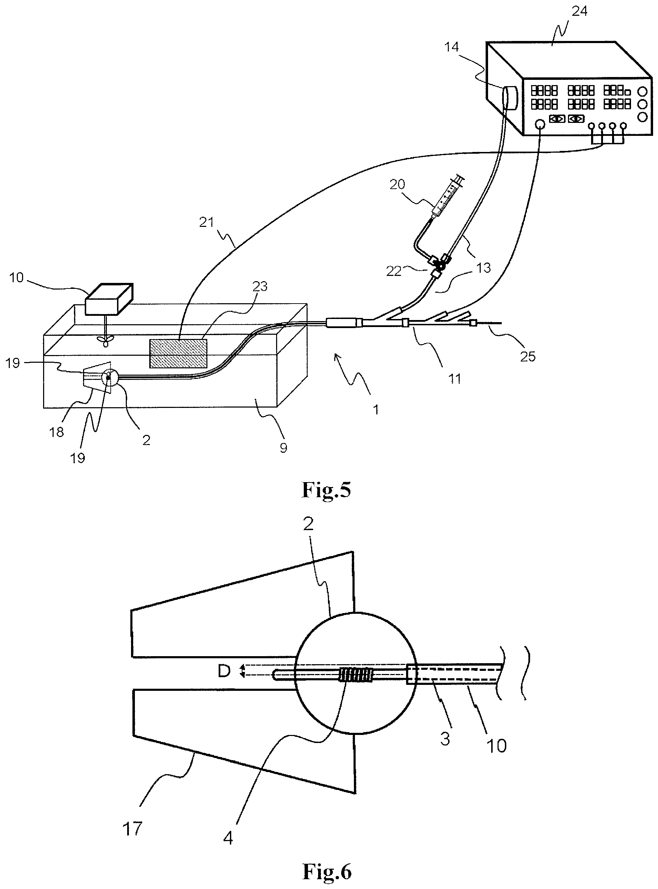

The branch path of the front handle 11f is connected to an extension tube 13 for injection/suction of the fluid into/from the lumen 17. The fluid is injected from a syringe 19 (illustrated in Fig. 5 explained below) connected to this extension tube 13, and the fluid is then injected into the balloon 2 through the lumen 17 between the outer cylinder shaft 10 and the inner cylinder shaft 3.

As a device arranged outside the balloon catheter 1, a vibrator 14 is arranged as agitation means for repeatedly suctioning and discharging a liquid inside the balloon 2 and thereby vibrating and agitating the liquid in order to stabilize the temperature inside the balloon 2. Since the agitation means vibrates and agitates the liquid, the liquid inside the balloon 2 is agitated, which equalizes the temperature inside and on the surface of the balloon.

An example of the vibrator 14 for vibrating the liquid in the balloon 2 is a device including a roller pump, a diaphragm pump, a bellows pump, a vane pump, a centrifugal pump, and a combination of a piston and a cylinder. The vibration frequency of the vibrator 14 is desirably 1 Hz to 5 Hz for repetitive suction/discharge of the liquid into/from the balloon so as to enable efficient agitation of the liquid in the balloon. However, another frequency can be set according to the design of the device.

During a procedure, a small amount of the physiological saline, about 100 ml per hour, is generally suctioned/discharged into/from the balloon 2 through the lumen 17, which exists between the inner cylinder shaft and the outer cylinder shaft, to prevent backflow of blood during the procedure.

The liquid used to vibrate the balloon is prepared by mixing the physiological saline containing 0.9 wt% of salt (sodium chloride) dissolved in water with a radiographic contrast agent. The amount of the liquid to be injected varies depending on the size of the balloon 2 and is usually from 5 mL to 30 mL.

One end of a power signal generated by the heating power supply means 24 illustrated in Fig. 1 is led to the heating coil electrode 4 through a conductive wire in a catheter cable, while one end of another power signal generated by the heating power supply means 24 is led to a return pad 23.

The heating power supply means 24 is preferably provided with: a current limiting function (for example, a function that automatically suppresses a source current of a metal-oxide semiconductor field-effect transistor (MOSFET) when the source current becomes 20 A or greater) for preventing the current of a rated value or greater from flowing to a power system device (such as a switching transistor, a transformer, or an inductor); or a safety function related to device overheat protection for avoiding a temperature increase of the power system device to a rated value or greater.

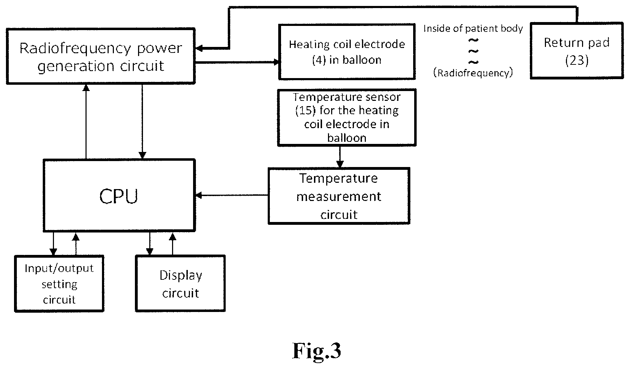

Fig. 3 is a block diagram of a temperature control system implemented by the heating power supply means 24. The practitioner makes settings required for the procedure via an input/output setting circuit including membrane switches or touch panel switches, and the settings are sent to a central processing unit (CPU) as the control section. A signal of the temperature sensor provided in the balloon 2 is converted from an analog voltage signal to a digital signal by a temperature measurement circuit and sent to the control section. In the control section, a control signal for a radiofrequency power generation circuit is generated by using the signal from the input/output setting circuit and the signal from the temperature measurement circuit, and the radiofrequency power generation circuit outputs the radiofrequency power.

The radiofrequency current generated by the radiofrequency power generation circuit is conducted through a patient body between the heating coil electrode 4 and the return pad attached to the back of the patient. The radiofrequency current flows through the balloon 2, which is filled with the liquid prepared by mixing the physiological saline containing 0.9 wt% of salt (sodium chloride) dissolved in water with an amount of the contrast agent equivalent to 20 to 50 volume% of the physiological saline, and the patient body. The mixing ratio of the liquids is not limited to these, and distilled water or the like may be mixed.

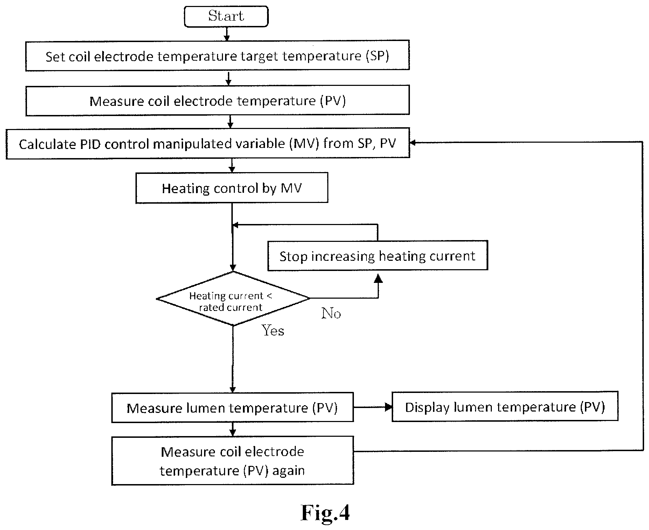

Fig. 4 is a flowchart illustrating temperature control operation of the ablation catheter system according to the first embodiment. As illustrated in the flowchart, the operator sets a target temperature (SP) of the coil electrode temperature using an operation switch on a front panel of the heating power supply means 24. It should be noted that the target temperature (SP) of the coil electrode temperature may be set to 70°C, for example, as a default value.

At the start, since the temperature (PV) by the coil electrode temperature sensor 15 indicates the internal body temperature of the patient, the temperature (PV) is lower than the target temperature (SP) (for example, 70°C) of the coil electrode temperature. Thus, control operation for the heating means is performed on the basis of an upper capacity limit of the heating power supply means 24.

When the control section continuously controls the heating means, in a process of calculating an manipulated variable (MV) from a deviation (E1) between an actual measured value (PV) of the coil electrode temperature based on the temperature sensor 15 for the coil electrode and an upper limit setting value (SP) of the coil electrode temperature, the manipulated variable was calculated by calculation based on a P parameter, which was set in advance, according to the P parameter.

Similarly, the control section calculated the manipulated variable by calculation based on a set I parameter according to the I-parameter, further calculated the manipulated variable by calculation based on a D parameter, which was set in advance, according to the D-parameter, and thereby determined the manipulated variable for executing PID control.

As the parameters, which were set in advance, 25% of the target temperature was used as a P value, 10 seconds was used as an I value, and 5 seconds was used as a D value. However, the P value, the I value, and the D value can each have any of various setting values according to the desired temperature control. In addition, as it is well known, a method in which the control section auto-tunes the PID prior to the temperature control and independently sets the P value, the I value, and the D value can also be employed.

Meanwhile, the output of the lumen temperature sensor 5, which displays the balloon surface temperature, is always displayed in the process of the heating control so as to inform the practitioner (operator) of the lumen temperature that reflects the balloon surface temperature.

Fig. 5 is a view illustrating external appearance of a simulated experimental system simulating actual use of the ablation catheter system. A tank 9 was filled with the physiological saline, in which 0.9 wt% of salt (sodium chloride) was dissolved in water, to simulate the body fluid, and the temperature of the physiological saline was maintained at a temperature similar to the body temperature (about 37°C) by a tank temperature controller 10. The operation in the first embodiment was tested using the simulated experimental system.

In the first embodiment, in estimating the positional relationship between the balloon and the shaft from the actual value of the time fluctuation in the output power from the heating power supply means 24, the estimation is made as to whether the positional relationship has a value corresponding to a coaxial state, an intermediate range state, or a non-coaxial state.

Here, the balloon 2 was applied substantially perpendicular to a pulmonary vein hole of a heart to be treated, and a state where the angle between the extended line of the agitation outer cylinder shaft 8 and the axial direction of the temperature sensor 15 for the coil electrode in the balloon 2 was within 10 degrees was defined as the coaxial state. In addition, a state where the angle between the extended line of the outer cylinder shaft 8 and the axial direction of the temperature sensor 15 for the coil electrode ranged from 10 to 20 degrees was defined as the intermediate range state, and a state where the angle between the extended line of the outer cylinder shaft 8 and the axial direction of the temperature sensor 15 for the coil electrode exceeded 20 degrees was defined as the non-coaxial state. However, this angular threshold is only used in the first embodiment, and an additional threshold for estimating the positional relationship between the balloon and the shaft may be provided according to an operative procedure, the size of the balloon, or the like.

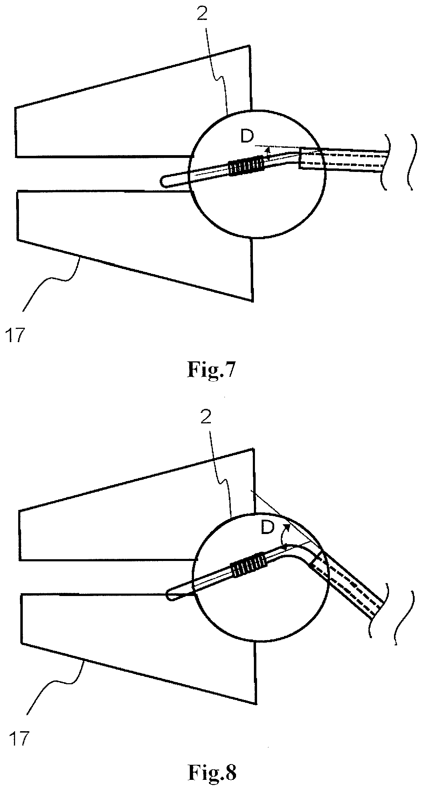

Here, Fig. 6 is a schematic view for explaining the relationship between the balloon 2 and the shaft 3 in the coaxial state, Fig. 7 is a schematic view for explaining the relationship between the balloon 2 and the shaft 3 in the intermediate range state, and Fig. 8 is a schematic view for explaining the relationship between the balloon 2 and the shaft 3 in the non-coaxial state. Figs. 6 to 8 illustrate cases where the angle between the extended line of the outer cylinder shaft 8 and the axial direction of the temperature sensor 15 for the coil electrode (denoted by symbol D in Figs. 6 to 8) is 0, 18, and 60 degrees, respectively. In the simulated experimental system in Fig. 5, the pulmonary vein hole portion of the heart, which corresponds to a treatment site, is a pseudo living body and balloon contact portion 19 provided in a pseudo living body 18.

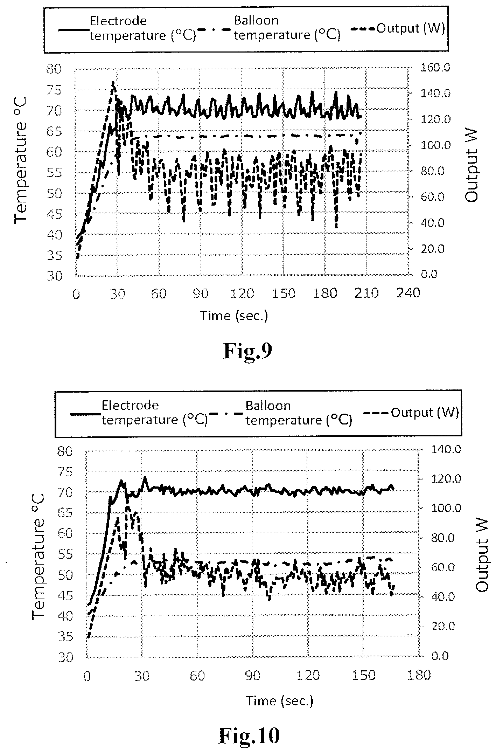

Fig. 9 indicates the temperature by the temperature sensor 15 for the coil electrode, the temperature by the lumen temperature sensor 5, and an actual measured value of the output power of the heating power supply means 24 in cases where the positional relationship between the balloon 2 and the shaft 3 is in the coaxial state and the temperature by the temperature sensor 15 for the coil electrode (a temperature near the heating coil electrode) is controlled to be 70°C in the simulated experimental system illustrated in Fig. 5. During ablation operation in the coaxial state, when the temperature by the temperature sensor 15 for the coil electrode (the temperature near the heating coil electrode) was controlled to be 70°C, the temperature by the temperature sensor 15 for the coil electrode was 70°C, the temperature by the lumen temperature sensor 5 was 61°C, and the actual measured value of the output power of the heating power supply means 24 was 75 W.

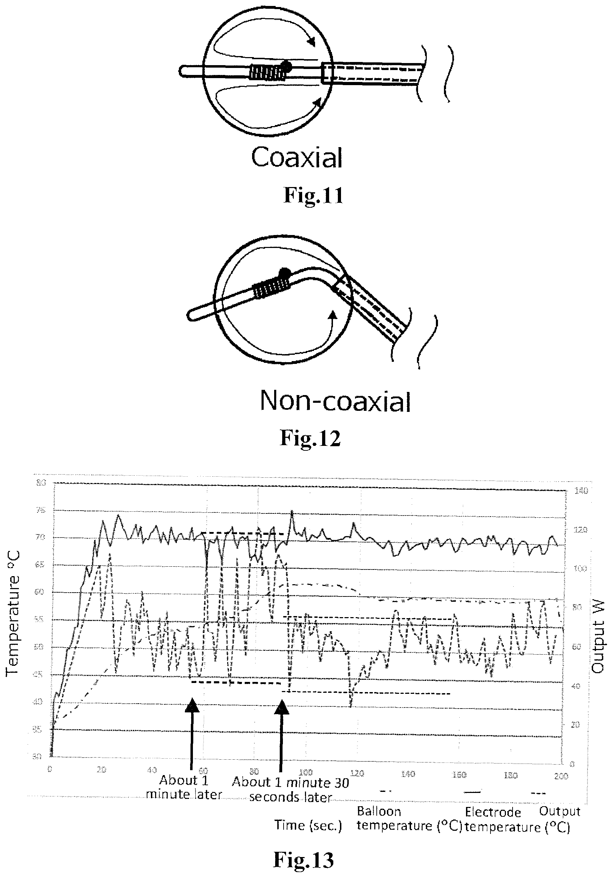

Next, Fig. 10 indicates the temperature by the temperature sensor 15 for the coil electrode, the temperature by the lumen temperature sensor 5, and the actual measured value of the output power of the heating power supply means 24 in cases where the positional relationship between the balloon 2 and the shaft 3 is in the non-coaxial state (Fig. 12) and the temperature by the temperature sensor 15 for the coil electrode is controlled to be 70°C during the ablation operation in the non-coaxial state.

In the coaxial state, as indicated in Fig. 9, at the start of the control when the temperature by the temperature sensor 15 for the coil electrode (the temperature near the heating coil electrode) was near the body temperature of the patient (about 37°C), heating was performed at an output power increase rate of 5 W/second for the output power of the output power supply means 24. Then, when the temperature by the temperature sensor 15 for the coil electrode (the temperature near the heating coil electrode) reached a control target temperature (70°C), the output power of the heating power supply means 24 was subjected to the PID control, and the temperature by the temperature sensor 15 for the coil electrode was controlled at 70°C ± 4°C. At this time, the output power of the heating power supply means 24 fluctuated in a range of about ±30 W.

Next, in the non-coaxial state, as indicated in Fig. 10, at the start of the control when the temperature by the temperature sensor 15 for the coil electrode (the temperature near the heating coil electrode) was near the body temperature of the patient (about 37°C), similar to the coaxial state, heating was performed at an output power increase rate of 5 W/second for the output power of the output power supply means 24. Then, when the temperature by the temperature sensor 15 for the coil electrode (the temperature near the heating coil electrode) reached the control target temperature (70°C), the output power of the heating power supply means 24 was subjected to the PID control, and the temperature by the temperature sensor 15 for the coil electrode was controlled at 70°C ± 2°C. At this time, the output power of the heating power supply means 24 fluctuated in a range of about ±20 W.

The following reason is inferred as a reason why the fluctuation is small in the non-coaxial state where the temperature by the temperature sensor 15 for the coil electrode (the temperature near the heating coil electrode) is controlled at 70°C ± 2°C when compared to the coaxial state where the temperature by the temperature sensor 15 for the electrode (the temperature near the heating coil electrode) is controlled at 70°C ± 4°C in the PID control. That is, as illustrated in a schematic view of Fig. 10, which illustrates a flow of the fluid in the balloon in the coaxial state, in the coaxial state, an agitation pump flow from the lumen 17 flows toward the coil electrode in the balloon, and the temperature sensor 15 for the coil electrode is exposed to the agitation flow. Meanwhile, as illustrated in a schematic view of Fig. 12, which illustrates the flow of the fluid in the balloon in the non-coaxial state, in the non-coaxial state, since the agitation pump flow from the lumen 17 is unevenly distributed in the balloon, the temperature sensor 15 for the coil electrode is less affected by the agitation flow.

The output power of the heating power supply means 24 is generated on the basis of the temperature by the temperature sensor 15 for the coil electrode. Accordingly, it is considered that the output power range is increased in the coaxial state with a large fluctuation in the temperature by the temperature sensor 15 for the coil electrode and that the output power range is reduced in the non-coaxial state with the small fluctuation in the temperature by the temperature sensor 15 for the coil electrode.

Fig. 12 indicates the actual measured values in cases where, during the procedure, the state was changed in an order of the non-coaxial state, the intermediate range state, and the coaxial state while the temperature by the temperature sensor 15 for the coil electrode, the temperature by the lumen temperature sensor 5, and the output power from the heating power supply means were measured and, after about 30 seconds, the state was changed in an order of the coaxial state, the intermediate range state, and the non-coaxial state.

About 1 minute after the start of heating of the balloon in Fig. 12, the state was changed from the non-coaxial state to the coaxial state. At this time, the surface temperature was gradually increased, and the time fluctuation in the output power of the heating power supply means 24 was increased.

Next, about 1 minute 30 seconds after the start of heating of the balloon in Fig. 12, the coaxial state was changed to the non-coaxial state. At this time, the increase in the surface temperature was stopped, and the time fluctuation in the output power of the heating power supply means 24 was reduced. Based on the operation of the heating power supply means 24 as described above, a calculation method for estimating the positional relationship between the balloon and the shaft was devised from the actual value of the time fluctuation in the output power from the heating power supply means 24.

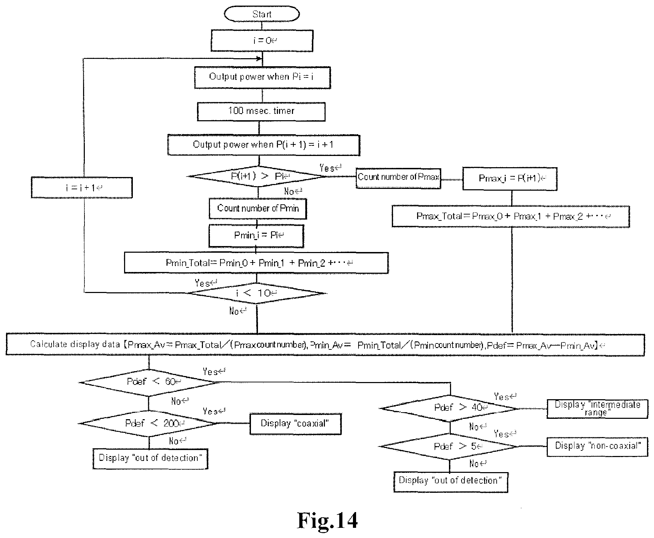

Fig. 13 is a flowchart of an estimation program of the coaxial state, the intermediate range state, and the non-coaxial state as a specific example of a program for estimating the positional relationship between the balloon and the shaft in the first embodiment. In operation for estimating the positional relationship between the balloon and the shaft, the control section in the first embodiment estimates estimated information on the positional relationship in the coaxial state, the intermediate range state, the non-coaxial state, or out of detection on the basis of the flowchart in Fig. 14. By executing the flowchart in Fig. 14, the positional relationship regarding whether the balloon and the shaft are in a linear state or, if not, to what extent the balloon and the shaft deviate from the linear state can be obtained as the estimated information on the positional relationship regarding the coaxial state, the intermediate range state, or the non-coaxial state. By obtaining the estimated information on the positional relationship regarding the coaxial state, the intermediate range state, or the non-coaxial state, the practitioner can be assisted with placement of the balloon against the tissue as intended.

First, a variable i is initialized to 0. Then, output power Pi of the heating power supply means 24 at the start of the program and output power P(i + 1) of the heating power supply means 24 after a lapse of 100 milliseconds are read. Pi and P(i+1) are compared, larger one thereof is assigned to Pmax_i, and smaller one thereof is assigned to Pmin_i.

Pmax_i and Pmin_i are updated once every 100 milliseconds and averaged for every 10 times of data obtainment (data acquisition) to obtain Pmax Av and Pmin_Av. In addition, Pdef that is a difference between Pmax_Av and Pmin_Av is calculated. This Pdef can be used as the actual value of the time fluctuation in the output power from the heating power supply means 24.

Accordingly, the output power of the heating power supply means 24 is measured every 100 milliseconds, the maximum value and the minimum value thereof are calculated for each measurement, and such calculations are repeated 10 times in 1 second. In the meantime, Pdef, which is the actual value of the time fluctuation in the output power from the heating power supply means 24, is obtained as a difference between the average value of the maximum values of the output power and the average value of the minimum values of the output power.

Thus, it is possible with Pdef to avoid an error in estimating the positional relationship between the balloon and the shaft due to sudden noise generated by power line superposition or radiation. In addition, since the determination on the estimated information on the positional relationship regarding the coaxial state, the intermediate range state, the non-coaxial state, or out of detection is updated every 1 second, it is possible to ensure accuracy and convenience as the device.

However, an updated cycle of Pmax_i and Pmin_i: 100 milliseconds and the number of the obtained pieces of the data (data acquisition) on the numerical value to be averaged, which is set to 10 in the above description, may be adjusted according to the device characteristics or the practitioner's preference for display.

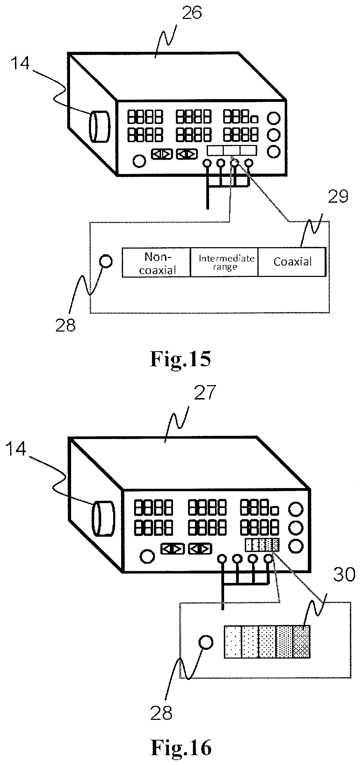

Fig. 15 is a view illustrating external appearance of the heating power supply means 24 in the first embodiment. The heating power supply means 24 in the first embodiment is provided with a display 29 that corresponds to the flowchart. In a manner to correspond to the estimation of the coaxial state, the intermediate range state, or the non-coaxial state as the positional relationship between the balloon and the shaft, the display 29 provided to a display panel of the heating power supply means 26 includes three segments such that the display 29 can display "coaxial" when Pdef is 60 w or greater, can display "intermediate range" when Pdef is 40 w to 60w, and can display "non-coaxial" when Pdef is 40 w or less. However, a threshold of Pdef, which is the obtained actual value of the time fluctuation, is not limited thereto, and another threshold can be set in advance in conjunction with the operative procedure and the balloon.

In a certain period from the start of the procedure (about 30 seconds from the start of the normal operation, depending on a power capacity of the heating power supply means 24), the output power of the heating power supply means 24 continues to be increased. In this case, the coaxial state, the intermediate range state, or the non-coaxial state can no longer be estimated from Pdef.

More specifically, the difference between Pmax_Av and Pmin_Av becomes extremely small and, in some cases, has a negative value. In the first embodiment, when Pdef was 5 w or less, it was determined that the estimation of the coaxial state, the intermediate range state, or the non-coaxial state from Pdef was difficult. Thus, as illustrated in the flowchart in Fig. 14, "out of detection" was displayed by lighting a point light-emitting LED in Fig. 15.

Meanwhile, in the course of the procedure, when the balloon moves away from the treatment site and the load on the heating power supply means 24 is reduced, the output power of the heating power supply means 24 continues to be reduced for a certain period (usually, such a situation rarely occurs and lasts about 5 to 10 seconds at the longest, depending on a period in which the balloon is away from the treatment site). In this case, the coaxial state, the intermediate range state, or the non-coaxial state can no longer be estimated from Pdef, either.

More specifically, the difference between Pmax_Av and Pmin_Av becomes extremely large. In the first embodiment, when Pdef was 200 w or greater, it was determined that the estimation of the coaxial state, the intermediate range state, or the non-coaxial state from Pdef was difficult. Thus, as illustrated in the flowchart in Fig. 14, "out of detection" was displayed by lighting the point light-emitting LED (32) in Fig. 15. The method for displaying "out of detection" is not limited to that by lighting the point light-emitting LED (32). For example, a method for blinking a part of the display 29 that displays "coaxial", "non-coaxial", or "intermediate range" is also effective. It should be noted that the display method in the display section may be display of an analog value instead of a discrete value such as "coaxial", "non-coaxial", "intermediate range", or "out of detection". This display can also be provided by changing a color in the display section.

The functions described so far enable to communicate, to the practitioner, the positional relationship between the balloon and the shaft, which has conventionally been extremely difficult but is important in the ablation treatment, by monitoring the time fluctuation in the output power (heating power) from the perspective of the coaxial state and the non-coaxial state and calculating the data obtained therefrom.

In the first embodiment, the threshold of the time fluctuation in the output power of the heating power supply means 24, which is recorded in the recording section, is used. The control section compares the actual value of the time fluctuation and the threshold of the time fluctuation in the output power from the heating power supply means 24 in the predetermined cycle, and thereby estimates the positional relationship between the balloon 2 and the outer cylinder shaft 8. However, alternatively, the recording section may record a conversion table used to convert the actual value of the time fluctuation in the output power from the heating power supply means 24 into the estimated information on the positional relationship between the balloon 2 and the outer cylinder shaft 8 on the basis of a plurality of parameters.

### (Second Embodiment)

As a second embodiment, an ablation catheter system is provided in which the display section 29 in the first embodiment is changed to a display section 30 that is segmented to five stages and thus can display the estimated information on the positional relationship between the balloon and the shaft in further detail and in which the operation of the control section to estimate the positional relationship between the balloon and the shaft is subdivided correspondingly. Fig. 16 is a view illustrating external appearance of heating power supply means 27 according to the second embodiment and the display section 30 provided thereto.

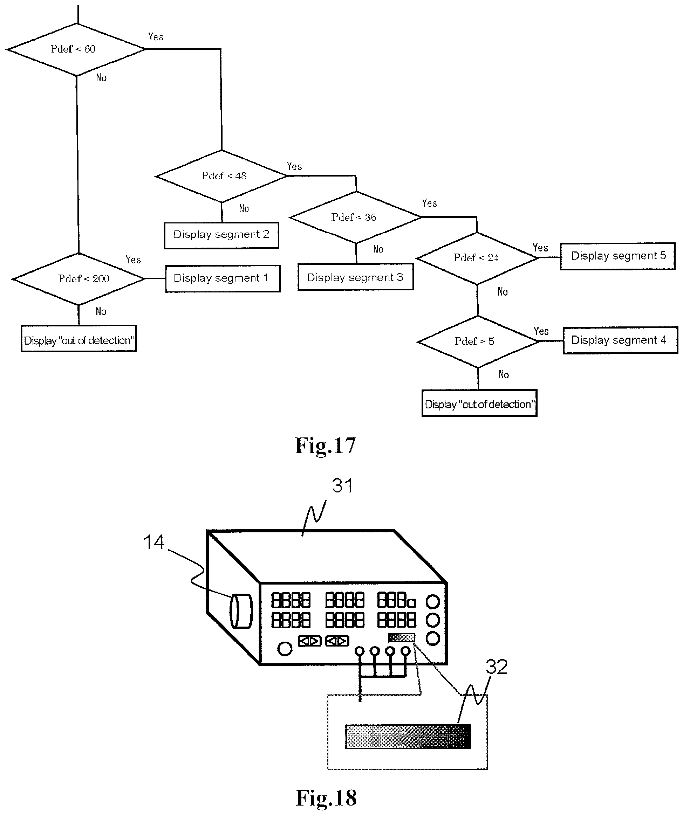

In addition, portions surrounded by dotted lines in the flowchart illustrated in Fig. 14 are replaced with a flowchart of a program for estimating the coaxial state, the intermediate range state, or the non-coaxial state in the second embodiment illustrated in Fig. 17, and the threshold that corresponds to Pdef as the actual value of the time fluctuation in the output power from the heating power supply means 24 is set finely. In this way, the segment of the display section 30 in Fig. 16 is divided into the five stages to further finely divide the coaxial state, the non-coaxial state, and the intermediate range state therebetween. Thus, it is possible to communicate, to the practitioner, the estimated information on the positional relationship between the balloon and the shaft, which is divided into five states, in more detail. More specifically, the coaxial state is achieved as a color becomes darker, and a degree of non-coaxiality is advanced as the color becomes lighter. It is also possible to display the coaxial state, the intermediate range state, or the non-coaxial state by color change.

In the flowchart illustrated in Fig. 17, after the operation to estimate the positional relationship between the balloon and the shaft, as control operation for display, a segment 1 is lit with Pdef being 60 w or greater, and a segment 2 portion is lit in addition to the segment 1 portion with Pdef being 60 w to 48 w.

Furthermore, when Pdef is 48 w to 36 w, a segment 3 portion is lit for display in addition to the segment 1 portion and the segment 2 portion. When Pdef is 36 w to 24 w, a segment 4 portion is lit for display in addition to the segment 1 portion, the segment 2 portion, and the segment 3 portion. When Pdef is 24 w or less, a segment 5 portion is lit for display in addition to the segment 1 portion, the segment 2 portion, the segment 3 portion, and the segment 4 portion. However, the threshold of Pdef, which is the obtained actual value of the time fluctuation, is not limited thereto, and another threshold can be set in advance in conjunction with the operative procedure and the balloon.

In cases where the output power of the heating power supply means 24 continues to be increased for a certain period, or where the output power of the heating power supply means 26 continues to be reduced for the certain period, similar to the first embodiment, the coaxial state, the intermediate range state, or the non-coaxial state as the positional relationship between the balloon and the shaft can no longer be estimated from Pdef. In the second embodiment, "out of detection" is displayed when Pdef is 200 w or greater or less than 5 w.

In the flowcharts for estimating the positional relationship between the balloon and the shaft in the first and second embodiments, a power cutoff value such as 60 w or 40 w depends on a design value of a control system. Thus, the above value is merely one example, and various settings are considered. Instead of the digital display method used in the first and second embodiments, an analog display method, such as display of the calculated value as is, may be adopted for the display section.

### (Third Embodiment)

As a third embodiment, an ablation catheter system is provided in which the display section 30 for displaying the coaxial state, the intermediate range state, or the non-coaxial state in the second embodiment is replaced with a display section 32 having more informative display means. Fig. 18 is a view illustrating external appearance of heating power supply means in the third embodiment. Since the display section 30 capable of displaying the segments 1 to 5 used in the second embodiment is changed to the display section 32 capable of displaying 10 segments, it is possible to correspond to a case where the positional relationship between the balloon and the shaft is finely estimated, and to visually divide the coaxial state, the non-coaxial state, and the intermediate range state therebetween in a more detailed manner by shading (gradation). In this way, the estimated information on the positional relationship between the balloon and the shaft, which is divided into 10 states, can be presented to the operator.

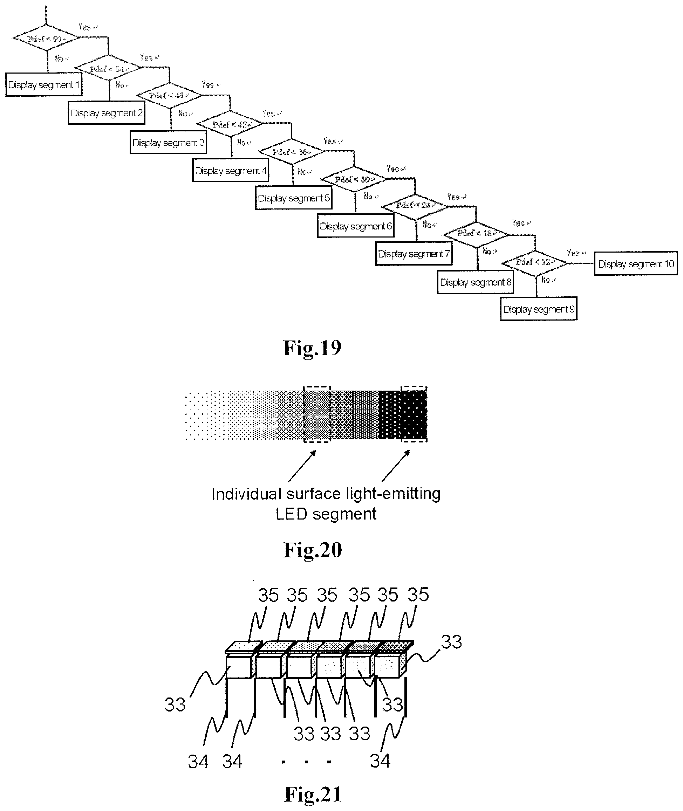

Fig. 19 is a specific flowchart of a program for estimating the coaxial state, the intermediate range state, or the non-coaxial state in the third embodiment to provide this display. In this case, a branch determination made by the comparison between Pdef, which is the actual value of the time fluctuation in the output power from the heating power supply means 24, and the set threshold is doubled from the five conditional determinations in the second embodiment to 10 determinations. This can increase strip-shaped LED surface display segments to 10, and the estimated information on the positional relationship between the balloon and the shaft can be presented to the operator by shading of the display section. Fig. 20 illustrates a configuration of a coaxial, non-coaxial display in this third embodiment. It should be noted that surface light-emitting electroluminescent elements (such as organic EL elements) can also be used for the strip-shaped LED surface display segments.

As a modified example for a case where it is difficult to produce fine shades with the strip-shaped surface display LED segments or the surface light-emitting electroluminescent elements, Fig. 21 illustrates an example of a display in which a white surface light-emitting LED and a color filter is combined. In this case, it is also considered to employ a method for displaying in the different shades by using a strip-shaped white surface display segment device and layering color films made of PET or the like on top thereof.

As Pdef deriving means for avoiding an estimation error of the coaxial state, the intermediate range state, or the non-coaxial state due to the sudden noise, in addition to the above method for using the average values of the maximum values and the minimum values, a method for using moving average values of the maximum values and the minimum values may be selected.

### (Fourth Embodiment)

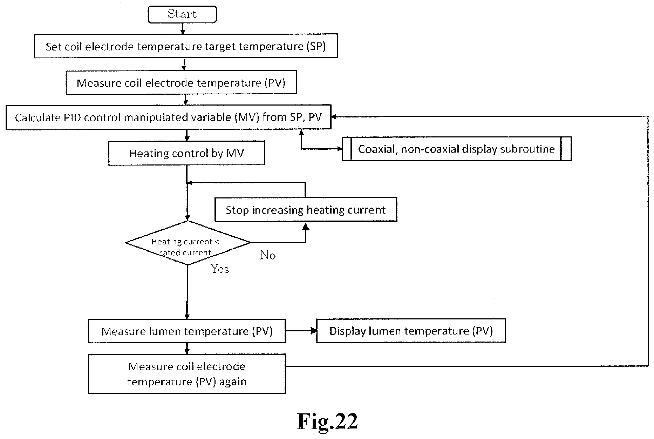

In a fourth embodiment, a balloon ablation catheter system is provided in which the obtainment of the actual value (Pdef) of the time fluctuation in the output power, which is the value changed over the treatment time, in addition to the calculation of the value of the output power from the heating power supply means 24 in the means for estimating the coaxial state, the intermediate range state, or the non-coaxial state as the positional relationship between the balloon and the shaft in the first embodiment is changed to obtainment of an actual value (Tdef) of the time fluctuation in the coil electrode temperature, which is a value changed over the treatment time, in addition to the calculation of the value of the temperature by the temperature sensor 15 for the coil electrode (the temperature near the heating coil electrode). Fig. 22 is a flowchart for estimating the positional relationship between the balloon and the shaft in the fourth embodiment.

The actual value of the time fluctuation in the temperature by the temperature sensor 15 for the coil electrode (the temperature near the heating coil electrode) shows a different value depending on the coaxial state, the intermediate range state, and the non-coaxial state. Thus, in the fourth embodiment, such a value is detected and displayed by a control system (program) related to the display and provided in the heating power supply means.

Operation in the fourth embodiment is described below. In the coaxial state, as illustrated in Fig. 10, at the start of the control when the temperature by the temperature sensor 15 for the coil electrode (the temperature near the heating coil electrode) was near the body temperature of the patient (about 37°C), heating was performed at an output power increase rate of 5 W/second for the output power of the output power supply means 24. Then, when the temperature by the temperature sensor 15 for the coil electrode (the temperature near the heating coil electrode) reached the control target temperature (70°C), the output power of the heating power supply means 24 was subjected to the PID control, and the temperature by the temperature sensor 15 for the coil electrode was controlled at 70°C ± 4°C. At this time, the temperature by the temperature sensor 15 for the coil electrode (the temperature near the heating coil electrode) fluctuated within the range of about ±4°C.

Next, in the non-coaxial state, as illustrated in Fig. 10, at the start when the temperature by the temperature sensor 15 for the coil electrode (the temperature near the heating coil electrode) was near the body temperature of the patient (about 37°C), similar to the coaxial state, heating was performed at an output power increase rate of 5 W/second for the output power from the output power supply means. Then, when the temperature by the temperature sensor 15 for the coil electrode (the temperature near the heating coil electrode) reached the control target temperature (70°C), the output power of the heating power supply means 24 was subjected to the PID control, and the temperature by the temperature sensor 15 for the coil electrode was controlled at 70°C ± 2°C. At this time, the temperature by the temperature sensor 15 for the coil electrode (the temperature near the heating coil electrode) fluctuated within the range of about ±2°C.

Compared to the case where, in the coaxial state, the temperature by the temperature sensor 15 for the coil electrode (the temperature near the heating coil electrode) is controlled at 70°C ± 4°C in the PID control, the temperature by the temperature sensor 15 for the coil electrode (the temperature near the heating coil electrode) is controlled at 70°C ± 2°C with a small fluctuation in the non-coaxial state. A reason for this is because it is assumed that, while, as illustrated in Fig. 11, the agitation pump flow from the lumen 17 flows toward the coil electrode in the balloon and the temperature sensor 15 for the coil electrode is exposed to the agitation flow in the coaxial state, the agitation pump flow from the lumen 17 is unevenly distributed in the balloon in the non-coaxial state as illustrated in Figure 12, and thus the temperature sensor 15 for the coil electrode is less affected by the agitation flow.

In Fig. 13 indicating the state change during the procedure, while the surface temperature was gradually increased from a time point at which the state was changed from the non-coaxial state to the coaxial state, the time fluctuation in the temperature by the temperature sensor 15 for the coil electrode (the temperature near the heating coil electrode) was increased.

In addition, in Fig. 13, when the state was changed from the coaxial state to the non-coaxial state, the increase in the surface temperature was stopped, and the time fluctuation in the temperature by the temperature sensor 15 for the coil electrode (the temperature near the heating coil electrode) was reduced.

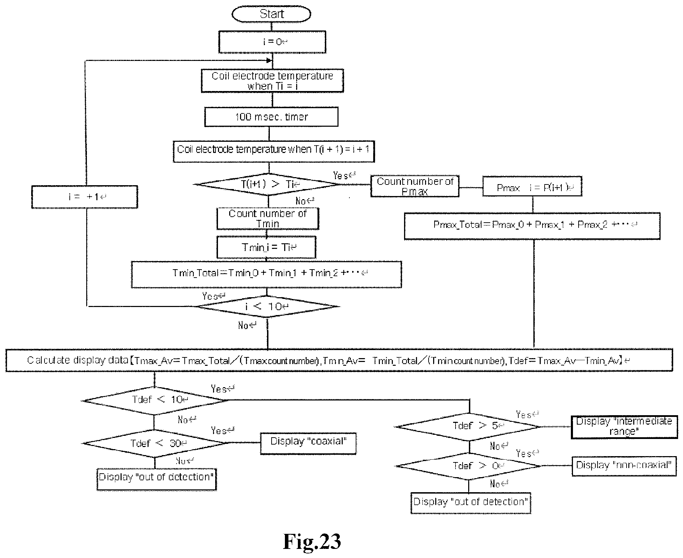

A calculation method for estimating the positional relationship between the balloon and the shaft from the time fluctuation in the temperature by the temperature sensor 15 for the coil electrode was devised on the basis of the relationship between the operation of the output power of the heating power supply means 24 and the temperature by the temperature sensor 15 for the coil electrode as described above. Such a method is illustrated in Fig. 23 as a flowchart in which the program for estimating the positional relationship between the balloon and the shaft is added to a flowchart of an overall control system for the heating power supply means. First, the variable i is initialized to 0, and a temperature Ti near the heating coil electrode at the start of the program is read. Ti and a coil electrode temperature T(i + 1) after 100 milliseconds are compared, larger one thereof is assigned to Tmax_i, and smaller one thereof is assigned to Tmin_i.

Tmax_i and Tmin_i are updated once every 100 milliseconds and averaged for every 10 times of data obtainment (data acquisition) to obtain Tmax_Av and Tmin_Av. In addition, a difference Tdef between Tmax_Av and Tmin_Av is calculated and used as the actual value of the time fluctuation in the temperature by the temperature sensor 15 for the coil electrode.

Similar to the first embodiment, the heating power supply means 24 in the fourth embodiment is provided with the display 29 that corresponds to the flowchart for estimating the positional relationship between the balloon and the shaft. In the manner to correspond to the estimation of the coaxial state, the intermediate range state, or the non-coaxial state, the display 29 provided to the display panel of the heating power supply means 24 can indicate "coaxial" when Tdef is 10°C or greater, can indicate "intermediate range" when Tdef is 5°C to 10°C, and can indicate "non-coaxial" when Tdef is 5°C or less. However, the threshold of Tdef, which is the actual value of the obtained time fluctuation, is not limited thereto, and another threshold can be set in advance in conjunction with the operative procedure or the balloon.

In cases where the temperature by the temperature sensor 15 for the coil electrode continues to be increased for a certain period, or the temperature by the temperature sensor 15 for the coil electrode continues to be reduced for the certain period, it is no longer possible to estimate, from Tdef, the coaxial state or the non-coaxial state as the positional state between the balloon and the shaft. In the fourth embodiment, "out of detection" is indicated when Tdef is 30°C or greater or 0°C or less.

Also, in the fourth embodiment, similar to the first to third embodiments, it is possible to inform the estimated information on the positional relationship between the balloon and the shaft more finely than "coaxial", "non-coaxial", and "intermediate range".

In the fourth embodiment, the threshold of the time fluctuation in the temperature by the temperature sensor 15 for the coil electrode, which is recorded in the recording section, is used. The control section compares the actual value of the time fluctuation and the threshold of the time fluctuation in the temperature by the temperature sensor 15 for the coil electrode in the predetermined cycle, and thereby estimates the positional relationship between the balloon 2 and the outer cylinder shaft 8. However, alternatively, the recording section may record a conversion table used to convert the actual value of the time fluctuation in the temperature by the temperature sensor 15 for the coil electrode into the estimated information on the positional relationship between the balloon 2 and the outer cylinder shaft 8 on the basis of a plurality of parameters.

### (Fifth Embodiment)

As a fifth embodiment, a balloon ablation catheter system is provided in which, instead of measuring the output power of the heating power supply means 24 in the means for estimating the coaxial state, the intermediate range state, or the non-coaxial state as the positional relationship between the balloon and the shaft in the first embodiment every 100 milliseconds, calculating the maximum value and the minimum value thereof for each measurement, and repeating such calculations 10 times to calculate Pdef on the basis of the difference between the average value of the maximum values of the output power and the average value of the minimum values of the output power, the output power of the heating power supply means 24 is measured every 100 milliseconds, and Pdef is calculated from a standard deviation of the output power obtained by each measurement.

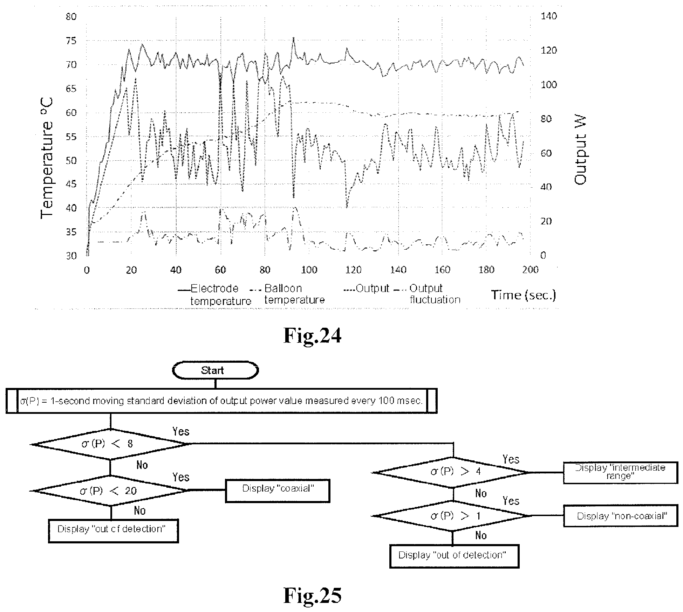

Next, operation in the fifth embodiment is described below. Fig. 24 is a graph illustrating a calculation result of the standard deviation of the output power in the same measurement as that in Fig. 13, and the calculation result of the standard deviation is used in the fifth embodiment. The standard deviation is a moving average value of the standard deviation that is calculated from 10 points of the output power measured every 100 milliseconds.

As illustrated in Fig. 24, the standard deviation of the output power fluctuates in response to the change in the estimated information on the positional relationship between the balloon and the shaft from the non-coaxial state, the coaxial state to the non-coaxial state in succession. More specifically, the standard deviation of the output power falls within a range of about ±4 w in the non-coaxial state, but the range expands to within a range of about ±8 w in the coaxial state.

Fig. 25 is a flowchart for estimating the coaxial state, the intermediate range state, or the non-coaxial state as the positional relationship between the balloon and the shaft in the fifth embodiment. It is possible to obtain the estimated information on the positional relationship, which is the coaxial state, the intermediate range state, or the non-coaxial state, from a value of σ(P) that is calculated in a subroutine for calculating the moving average value of the standard deviation calculated from 10 points of the output power measured every 100 milliseconds. Alternatively, the value of the standard deviation may be displayed as is.

However, the updated cycle: 100 milliseconds and the number of the obtained pieces of the data on the numerical value to be averaged (data acquisition), which is set to 10 in the above description, may be adjusted according to the device characteristics or the practitioner's preference for display.

### (Sixth Embodiment)

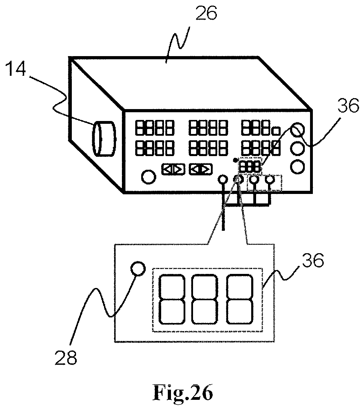

As a sixth embodiment, a balloon ablation catheter system is configured in which the control section does not estimate the positional relationship between the balloon and the shaft, and only obtains the actual value (Pdef) of the time fluctuation in the output power, which is a value changed in the course of the treatment time, in addition to the calculation of the value of the output power from the heating power supply means 24, or only obtains the actual value (Tdef) of the time fluctuation in the coil electrode temperature, which is a value changed in the course of the treatment time, in addition to the calculation of the value of the temperature by the temperature sensor 15 for the coil electrode (the temperature near the heating coil electrode), and in which the display section displays the actual value (Pdef) of the time fluctuation in this output power or the actual value (Tdef) of the time fluctuation in the coil electrode temperature. Fig. 26 illustrates an example of external appearance of heating power supply means that includes a display for displaying the positional relationship between the balloon and the shaft in a numerical value. This ablation catheter can be controlled in the same manner as those in the first to fifth embodiment until the operation for estimating the positional relationship between the balloon and the shaft.

In this case, the actual value (Pdef) of the time fluctuation in the obtained output power or the actual value (Tdef) of the time fluctuation in the obtained coil electrode temperature is displayed as is as the actual numerical value, such as Arabic numerals, on the display section. Thus, the practitioner can estimate the positional state between the balloon and the shaft by himself or herself on the basis of the numeral value.

### INDUSTRIAL APPLICABILITY

The present invention can further improve the convenience of the practitioner and effectiveness of treatment in the balloon catheter system for the treatment of arrhythmia such as atrial fibrillation, endometriosis, cancer, and other conditions.

### DESCRIPTION OF SYMBOLS

1: balloon catheter, 2: balloon, 3: inner cylinder shaft, 4: heating coil electrode, 5: lumen temperature sensor, 6: power supply lead wire, 7: lumen temperature sensor lead wire, 8: outer cylinder shaft, 9: tank, 10: tank temperature controller, 11: handle (11f: front handle, 11r: rear handle), 12: connector, 13: extension tube, 14: vibrator, 15: temperature sensor for coil electrode, 16: temperature sensor lead wire for coil electrode, 17: lumen, 18: pseudo living body, 19: pseudo living body and balloon contact portion, 20: syringe, 21: return pad cable, 22: three-way stopcock, 23: return pad, 24: heating power supply means, 25: guide wire, 26: heating power supply means, 27: heating power supply means, 28: point light-emitting LED, 29: display, 30: five-segment display, 31: heating power supply means, 32: gradation-type display (ten-segment display), 33: white surface light-emitting LED, 34: shielding plate, 35: color filter, 36: display displaying positional relationship between balloon and shaft in numerical value

## Claims

1. An ablation catheter system, comprising:
a shaft having a lumen through which fluid can be delivered;
a balloon attached to the distal end of the shaft;
heating means arranged in the balloon;
heating power supply means for supplying power to the heating means;
a temperature sensor arranged near the heating means; and
a control section for estimating a positional relationship between the balloon and the shaft,
wherein the control section calculates an actual value of a time fluctuation in output power from the heating power supply means or an actual value of a time fluctuation in a temperature by the temperature sensor, and estimates the positional relationship between the balloon and the shaft from the obtained actual value.

2. The ablation catheter system of claim 1, comprising a recording section for recording a threshold of the time fluctuation in the output power from the heating power supply means or a threshold of the time fluctuation in the temperature by the temperature sensor,
wherein the control section compares the threshold of the time fluctuation and the actual value of the time fluctuation in a predetermined cycle to estimate the positional relationship between the balloon and the shaft.

3. The ablation catheter system of claim 1, comprising a recording section for recording a conversion table used to convert the actual value of the time fluctuation in the output power from the heating power supply means or the time fluctuation in the temperature by the temperature sensor into the positional relationship between the balloon and the shaft,
wherein the control section estimates the positional relationship between the balloon and the shaft on the basis of the time fluctuation and the conversion table.

4. The ablation catheter system of any one of claims 1 to 3, comprising a display section for displaying estimated information on the positional relationship between the balloon and the shaft.

5. The ablation catheter system of claim 4, wherein the display section displays the positional relationship between the balloon and the shaft with a collection of surface light-emitting elements.

6. An ablation catheter system, comprising:
a shaft having a lumen through which fluid can be delivered;
a balloon attached to the distal end of the shaft;
heating means arranged in the balloon;
heating power supply means for supplying power to the heating means;
a temperature sensor arranged near the heating means;
a control section for calculating an actual value of a time fluctuation in output power from the heating power supply means or an actual value of a time fluctuation in a temperature by the temperature sensor; and
a display section for outputting the actual value of the time fluctuation.
